# FASCICULE DE BREVET EUROPEEN

(11) **EP 4 541 399 B1**
(45) Date de publication et mention de la délivrance du brevet: **29.07.2026**
(21) Numéro de dépôt: 25156134.6
(22) Date de dépôt: 10.02.2022
(51) Int. Cl.: A61M 16/00

(54) **APPAREIL À TOUX METTANT EN OEUVRE UNE ANIMATION VIDÉO POUR AIDER LE PATIENT À EFFECTUER SON TRAITEMENT, EN PARTICULIER EN MODE INEX**
HUSTENGERÄT MIT VIDEOANIMATION ZUR UNTERSTÜTZUNG DER BEHANDLUNG, INSBESONDERE IM INEX-MODUS
COUGH APPARATUS USING VIDEO ANIMATION TO ASSIST PATIENT TREATMENT, IN PARTICULAR IN INEX MODE

(30) Priorité: 23.04.2021 FR 2104247
(43) Date de publication de la demande: 23.04.2025
(62) Demande divisionnaire de: 23204132.7
(73) Titulaire: Air Liquide Medical Systems, 92182 Antony Cedex (FR); EOVE, 64000 Pau (FR)
(72) Inventeur: LEROUX, Jean, 92182 Antony (FR); GALBRUN, Marie-Amédée, 64000 Pau (FR)
(74) Mandataire: Air Liquide

(56) Documents cités:
- EP-A1- 3 622 991
- EP-B1- 3 622 992
- US-A1- 2007 199 566

## Description

La présente invention concerne un appareil d'assistance à la toux ou appareil à toux permettant d'opérer des insufflations et des exsufflations de gaz à des patients souffrant de troubles respiratoires nécessitant une aide à l'évacuation des sécrétions pulmonaires, lequel peut être utilisé facilement par une personne n'ayant pas ou peu de connaissances médicales, typiquement une auxiliaire de vie, une personne de l'entourage familial du patient, encore appelés les aidants.

Les dispositifs médicaux appelés « assistants de toux », « appareils d'assistance à la toux », « appareil à toux » ou analogue, sont des appareils complémentaires aux respirateurs médicaux, qui permettent de générer une pression gazeuse contrôlée afin d'aider un patient à tousser, expectorer et se désencombrer.

Le principe d'un assistant de toux est d'aider à la mobilisation et à l'expectoration des sécrétions bronchiques en gonflant les poumons avec une pression positive puis en appliquant une pression négative pour faciliter le déplacement des mucosités. De tels appareils assistants de toux sont notamment décrits par EP-A-3622991, EP-A-3622992 et EP-A-862922.

Les dispositifs d'assistance à la toux comprennent généralement une turbine motorisée, aussi appelée compresseur ou micro-soufflante, tournant à vitesse fixe ou variable, couplée à une ou plusieurs électrovannes permettant d'orienter le flux d'air depuis la turbine vers le patient, lors des phases d'insufflation de gaz, puis depuis le patient vers la turbine, lors des phases d'exsufflation. Le patient est donc soumis à une alternance de phases d'insufflation (IN) et d'exsufflation (EX) de gaz.

Lors des phases d'insufflation, la sortie de turbine est connectée fluidiquement au patient, alors que, lors des phases d'exsufflation, l'entrée d'air de la turbine est connectée fluidiquement au patient afin d'assurer une aspiration. Des capteurs de pression et de débit coopérant avec des moyens de pilotage permettent de gérer les différentes phases en fonction des réglages choisis, en particulier des modes ventilatoires.

Un oscillateur de gaz peut également être prévu pour augmenter la capacité du dispositif à mobiliser les sécrétions pulmonaires ou bronchiques des patients traités.

La manipulation de ces appareils est habituellement confiée à un professionnel de santé, principalement les kinésithérapeutes. Ceci implique donc un déplacement systématique du professionnel de santé au domicile de chaque patient, jusqu'à plusieurs fois par jour, lorsque plusieurs séances quotidiennes sont nécessaires. De plus, le traitement du patient ne se fait pas forcément au meilleur moment pour lui car il dépend essentiellement du passage du professionnel de santé.

N'étant ni pratique ni efficient, il serait souhaitable de pouvoir déclencher une séance d'utilisation de l'appareil à toux au meilleur moment pour le patient de manière à la rendre plus efficace et par ailleurs à réduire considérablement le stress du patient et à éviter des déplacements pouvant être fréquents du personnel de santé.

De plus, il peut être compliqué pour certains patients, notamment les patients pédiatriques (enfants...), d'utiliser correctement l'appareil à toux lorsqu'un kinésithérapeute ou analogue n'est pas présent pour les aider, car ils doivent réussir à synchroniser leur respiration avec les phases d'insufflation et d'exsufflation de gaz par la turbine de l'appareil à toux, pour que le traitement soit efficace.

On connait par ailleurs US-A-2007/0199566 qui enseigne un ventilateur médical permettant de réaliser des insufflations et exsufflations de gaz à un patient. Un afficheur graphique permet d'afficher une représentation des poumons du patient dont la taille augmente ou diminue en fonction de mesures d'un paramètre thoracique du patient, ce qui permet de suivre les inspirations et expirations du patient. Toutefois, l'affichage du mouvement des poumons reflète uniquement la manière de respirer du patient mais ne constitue pas une aide permettant au patient de synchroniser sa respiration avec les phases d'insufflation et d'exsufflation de gaz par la turbine de l'appareil à toux, étant donné que cet affichage résulte de mesures opérées sur le patient.

De là, le problème est de proposer un appareil d'assistance à la toux ou appareil à toux pouvant fonctionner simplement et être mis en œuvre par une personne moins formée qu'un kinésithérapeute, par exemple une auxiliaire de vie, une personne de l'entourage familial du patient... de sorte à pouvoir déclencher chez un patient, c'est-à-dire débuter, une séance d'utilisation de l'appareil à toux, au moment le plus opportun pour le patient considéré, et sans avoir à faire venir spécialement un professionnel de santé qualifié de type kinésithérapeute ou analogue, tout en obtenant un traitement aussi efficace qu'en présence d'un tel kinésithérapeute et/ou en rendant le patient adhérent ou plus adhérent à son traitement.

Dit autrement, on souhaite pouvoir disposer d'un appareil à toux permettant à un patient de synchroniser simplement sa respiration avec les phases d'insufflation et d'exsufflation de gaz par la turbine de l'appareil à toux afin d'aboutir à un traitement efficace.

L'invention concerne alors un appareil à toux pour opérer des insufflations et exsufflations de gaz à un patient, comprenant une turbine motorisée en communication fluidique avec un circuit de gaz comprenant une ligne d'insufflation et une ligne d'exsufflation, un écran d'affichage, et des moyens de pilotage comprenant un microprocesseur, configurés pour commander la turbine et/ou un affichage sur l'écran d'affichage, et dans lequel les moyens de pilotage sont configurés pour afficher sur l'écran d'affichage, une animation vidéo mettant en œuvre une première représentation graphique symbolisant au moins les phases d'insufflation et d'exsufflation de gaz et une seconde représentation graphique symbolisant le patient.

De plus, il comprend en outre des moyens de mémorisation configurés pour mémoriser la vidéo d'animation.

Dans ledit appareil à toux :
- les dimensions des première et seconde représentations graphiques affichées sur l'écran d'affichage varient en corrélation avec les phases d'insufflation et d'exsufflation de gaz par la turbine, et de manière inverse les unes des autres (e.g. l'une à une taille qui augmente, quand celle de l'autre diminue), de manière à d'aider le patient à synchroniser (i.e. calquer) sa respiration avec les phases d'insufflation et d'exsufflation de gaz opérées par la turbine de l'appareil à toux, c'est-à-dire dire en calquant ses inspirations et expirations sur les variations de dimensions des représentations graphiques affichées sur l'écran d'affichage.
- la turbine comprend un moteur électrique, la ligne d'insufflation étant raccordée fluidiquement à une sortie de gaz de la turbine et la ligne d'exsufflation étant accordée fluidiquement à une entrée de gaz de la turbine, et
- les moyens de pilotage sont configurés pour commander la turbine de manière à déclencher cycliquement une phase d'insufflation et une phase d'exsufflation, telles que :
   - pendant chaque phase d'insufflation, la turbine génère une pression positive (P+) comprise entre 5 et 70 mbar (pression relative par rapport à la pression atmosphérique) et
   - pendant chaque phase d'exsufflation, la turbine génère une pression négative (P-) comprise entre 0 et -70 mbar (pression relative par rapport à la pression atmosphérique).

Préférentiellement, les moyens de pilotage sont aussi configurés pour afficher sur l'écran d'affichage, ladite animation vidéo dans laquelle la forme et/ou l'aspect des première et/ou seconde représentations graphiques affichées sur l'écran d'affichage varient également en corrélation avec les phases d'insufflation et d'exsufflation de gaz par la turbine, de manière à d'aider le patient à synchroniser (i.e. calquer) sa respiration avec les phases d'insufflation et d'exsufflation de gaz opérées par la turbine de l'appareil à toux, en particulier en se basant sur les variations de forme et/ou d'aspect, et de dimensions des représentations graphiques affichées sur l'écran d'affichage.

Selon le cas, l'appareil à toux divulgué peut comprendre l'une ou plusieurs des caractéristiques techniques suivantes :
- les moyens de pilotage sont configurés pour afficher sur l'écran d'affichage, ladite animation vidéo dans laquelle la forme et l'aspect des première et seconde représentations graphiques varient en corrélation avec les phases d'insufflation et d'exsufflation de gaz par la turbine.
- l'animation vidéo comprend une pluralité d'images successives.
- les moyens de pilotage sont configurés pour afficher sur l'écran d'affichage, une animation vidéo dans laquelle la forme, l'aspect et/ou les dimensions des première et seconde représentations graphiques affichées sur l'écran d'affichage varient de manière synchronisée l'une à l'autre.
- les moyens de pilotage sont configurés pour commander, en réponse à une activation par l'utilisateur d'une touche de démarrage/arrêt de traitement, un démarrage de la turbine et simultanément de l'animation vidéo, c'est-à-dire un début de traitement du patient.
- les moyens de pilotage sont configurés pour stopper, en réponse à une activation par l'utilisateur de moyens de démarrage/arrêt de traitement, un arrêt de la turbine et simultanément de l'animation vidéo, c'est-à-dire un arrêt de traitement du patient.
- les moyens de démarrage/arrêt de traitement comprennent une touche de démarrage/arrêt unique (i.e. une seule et même touche) commandant le démarrage et l'arrêt de la turbine et simultanément de l'animation vidéo.
- alternativement, les moyens de démarrage/arrêt de traitement comprennent deux touches distinctes, l'une commandant le démarrage et l'autre l'arrêt de la turbine et simultanément de l'animation vidéo.
- la (ou les) touche tactile est affichée sur l'écran d'affichage, c'est-à-dire que l'écran d'affichage est configuré pour afficher la (ou les) touche tactile.
- la (ou les) touche tactile coopère avec les moyens de pilotage.
- les variations de dimensions de la première représentation graphique et/ou de la seconde représentation graphique se font de manière progressive entre le début et la fin des phases d'insufflation et d'exsufflation de gaz et/ou des phases inspiratoire et expiratoire du patient.
- la première représentation graphique représente ou comprend un objet contenant du gaz, c'est-à-dire symbolisant la source de gaz, par exemple un ballon de baudruche.
- la seconde représentation graphique représente ou comprend un animal ou un personnage, par exemple un caméléon ou tout autre animal, c'est-à-dire un animal ou un personnage symbolisant le patient qui suit le traitement par insufflation et exsufflation de gaz.
- les moyens de pilotage sont configurés pour débuter le déroulé de l'animation vidéo affichée après activation par l'utilisateur des moyens de démarrage de l'insufflation/exsufflation de gaz.
- les dimensions (donc aussi la forme générale) des première et seconde représentations graphiques affichées sur l'écran d'affichage varient de manière inverse l'une de l'autre, c'est-à-dire que les dimensions de la seconde représentation graphique augmentent lorsque les dimensions de la première représentation graphique diminuent, et inversement (i.e. leur variation de dimensions et/ou de forme sont corrélées l'une à l'autre).
- la première représentation graphique symbolise les phases d'insufflation et d'exsufflation de gaz de l'appareil à toux, et éventuellement une phase de pause séparant une phase d'exsufflation de la phase d'insufflation suivante, et pendant laquelle du gaz n'est ni insufflé, ni exsufflé.
- la seconde représentation graphique symbolise au moins les phases d'inspiration et d'expiration du patient et éventuellement la phase de pause pendant laquelle le patient ni n'inspire, ni n'expire de gaz.
- les moyens de pilotage sont configurés pour commander la turbine de manière à insuffler du gaz au patient, typiquement de l'air.
- les moyens de pilotage sont configurés pour commander la turbine de manière à exsuffler du gaz du patient.
- l'écran d'affichage est configuré pour afficher la vidéo d'animation en couleurs ou alternativement en noir et blanc.
- l'appareil comprend en outre des moyens de mémorisation configurés pour mémoriser la pluralité d'images formant la vidéo d'animation, par exemple une mémoire informatique de type EEPROM ou analogue.
- l'affichage de la vidéo d'animation est opéré en temps réel pendant les phases d'insufflation et d'exsufflation de gaz.
- les moyens de sélection de mode de ventilation comprennent une ou des touches tactiles.
- l'appareil comprend en outre des moyens de sélection d'un mode de ventilation choisi parmi les modes INEX et IPPB.
- les moyens de sélection de mode de ventilation sont configurés pour sélectionner un ensemble de paramètres ou préréglages ventilatoires, aussi appelé programme de ventilation, adapté à la mise en œuvre d'une ventilation en mode de ventilation INEX ou IPPB.
- le ou les préréglages ventilatoires de chaque mode de ventilation sont fixés ou choisis par un personnel soignant.
- le ou les préréglages de chaque mode de ventilation sont mémorisés.
- l'écran d'affichage est configuré pour afficher la ou les touches tactiles configurées pour permettre à un utilisateur de sélectionner un mode de ventilation désiré choisi parmi les modes INEX et IPPB.
- le mode INEX correspond à une alternance de phases insufflatoire et exsufflatoire de gaz, éventuellement espacées par une phase de pause, avec insufflation de gaz à pression positive haute assez élevée, par exemple d'au moins 5 mbar, de préférence d'au moins 15 à 20 mbar, par exemple de l'ordre de 30 bar, pendant une durée d'insufflation courte, par exemple de 1 à 3 sec, suivi d'une exsufflation de gaz par mise en dépression (i.e. pression négative), par exemple de l'ordre de -30 mbar, des poumons et de la cage thoracique du patient pour provoquer la mobilisation et l'expulsion/extraction des sécrétions pulmonaires et/ou bronchiques La pression positive haute, la pression négative et les durées d'insufflation et/ou d'exsufflation sont réglables.
- le mode IPPB correspond à une alternance de phases insufflatoire et exsufflatoire de gaz, éventuellement espacées par une phase de pause, avec insufflation de gaz à débit faible, par exemple compris entre 5 et 100 L/min, par exemple entre 10 et 15 L/min, jusqu'à atteindre une pression de consigne maximale ou une durée d'insufflation maximale, typiquement une pression de consigne maximale entre 10 et 50 mbar, par exemple de l'ordre de 30 à 40 mbar, pendant une durée d'insufflation variable (pouvant atteindre de l'ordre de 10 secondes) et avec fourniture d'un volume de gaz variable, en fonction de l'élasticité des poumons et de la cage thoracique du patient, suivi d'une exsufflation de gaz sans mise en dépression (pression négative) des poumons et de la cage thoracique du patient, par exemple pendant une durée de 1 à 3 secondes, pour provoquer un recrutement alvéolaire et/ou une mobilisation des sécrétions et l'expectoration. Les pressions (i.e. positive et/ou négative) et/ou la durée d'insufflation maximale et/ou d'exsufflation sont réglables.
- le déroulé de l'affichage de la vidéo d'animation est synchronisé avec les durées d'insufflation et d'exsufflation, lors des phases d'insufflation ou d'exsufflation de gaz, en particulier en mode INEX.
- les durées d'insufflation et d'exsufflation sont réglables et/ou paramétrables.
- il comprend en outre des moyens de réglage de la durée d'insufflation et/ou d'exsufflation et/ou de la pression positive d'insufflation et/ou négative d'exsufflation, en particulier en mode INEX.
- la vidéo d'animation comprend plusieurs images différentes successives comprenant les première et seconde représentations graphiques dont la forme, l'aspect et/ou les dimensions affichées sur l'écran d'affichage varient en corrélation avec les durées d'insufflation et d'exsufflation lors des phases d'insufflation et d'exsufflation de gaz, en particulier en mode INEX.
- l'écran d'affichage comprend en outre des moyens de sélection d'un sous-mode de fonctionnement, de préférence une (ou des) touche tactile affichée sur l'écran, permettant de sélectionner un sous-mode de fonctionnement choisi parmi les modes automatique (AUTO) et manuel.
- les moyens de pilotage sont configurés pour afficher sur l'écran d'affichage et démarrer l'animation vidéo uniquement après sélection par l'utilisateur d'un préréglage correspond à un sous-mode automatique (AUTO) du mode de ventilation INEX, c'est-à-dire une ventilation INEX AUTO.
- les moyens de mémorisation sont configurés pour enregistrer, i.e. mémoriser, les préréglages (i.e. ensemble de paramètres) correspondant aux modes INEX et IPPB, voire d'autres préréglages, notamment un préréglage INEX AUTO.
- les préréglages comprennent des paramètres de ventilation adaptés aux modes INEX et IPPB, ou d'autres modes, y compris INEX AUTO
- les préréglages comprennent en particulier des valeurs de pression inspiratoire et expiratoire, des temps inspiratoire et expiratoire, un nombre de cycles de ventilation, une durée de pause, un niveau de PEP (pression expiratoire positive), les critères des oscillations, un seuil de déclenchement ou 'trigger' ou autre.
- les préréglages sont fixés par un personnel soignant, par exemple un médecin ou un kinésithérapeute, et mémorisés au sein des moyens de mémorisation préalablement au déroulé d'une séance de thérapie.
- les moyens de pilotage sont en outre configurés pour retrouver au sein des moyens de mémorisation, au moins un préréglage donné adapté à la mise en œuvre d'un mode de ventilation INEX ou IPPB en réponse à une sélection par un utilisateur, via les moyens de sélection de mode de ventilation, d'un desdits modes de ventilation INEX ou IPPB, y compris en sous-mode AUTO, i.e. INEX AUTO notamment.
- il comprend en outre une touche permettant de sélection ou rajouter un (ou des) cycle de ventilation supplémentaire, préférence une touche tactile affichée sur l'écran.
- un cycle de ventilation comprend une phase d'insufflation, une phase d'exsufflation et éventuellement une phase de pause. Typiquement, la durée d'un cycle est de l'ordre de 5 à 12 secondes.
- il comprend en outre des moyens d'arrêt de cycle, typiquement une touche tactile affichée sur l'écran d'affichage, permettant de stopper un cycle de ventilation en cours, c'est-à-dire de l'arrêter avant la fin du cycle.
- il comprend une touche de mise en route (ON) ou arrêt (OFF) de l'appareil, de préférence une touche tactile affichée sur l'écran.
- il comprend au moins une touche pour régler ou sélectionner un ou des préréglages adaptés aux modes de ventilation INEX et IPPB, de préférence une touche tactile affichée sur l'écran.
- il comprend en outre une touche pour sélectionner un préréglage du sous-mode de fonctionnement automatique (AUTO) en mode de ventilation INEX, c'est-à-dire une ventilation en mode INEX AUTO, de préférence une touche tactile affichée sur l'écran.
- il comprend en outre une touche d'arrêt de cycle pour stopper un cycle de ventilation en cours, c'est-à-dire avant la fin du cycle, de préférence une touche tactile affichée sur l'écran.
- il comprend en outre une touche de démarrage/arrêt de traitement pour démarrer et/ou stopper le fonctionnement de la turbine, c'est-à-dire pour commencer ou arrêter des insufflations et exsufflations de gaz, en particulier pour commencer ou arrêter une séance de traitement du patient, de préférence une touche tactile affichée sur l'écran.
- il comprend en outre une touche de menu pour accéder à différents menus ou sélections, de préférence une touche tactile affichée sur l'écran.
- l'écran d'affichage est configuré pour afficher en outre un débit maximal de gaz (en L/min par exemple), un volume courant de gaz (en mL par exemple) et/ou d'autres informations ou paramètres.
- les moyens de pilotage sont configurés pour commander la turbine de manière à délivrer du gaz pendant les phases d'insufflation et d'exsufflation de gaz au patient.
- il comprend une carcasse rigide, c'est-à-dire une coque externe.
- la turbine motorisée, la ligne d'insufflation, la ligne d'exsufflation et les moyens de pilotage sont agencés dans la carcasse.
- l'écran d'affichage est porté par la carcasse.
- l'écran d'affichage est fixé, i.e. solidarisé, de manière détachable ou non détachable à la carcasse.
- il comprend une interface homme-machine (IHM), aussi appelée interface graphique utilisateur (IGU), configurée pour permettre à un utilisateur d'opérer une ou des sélections ou des choix, de rentrer ou d'ajuster/modifier une ou des valeurs de consigne, notamment des valeurs de pression haute et basse, de temps des différentes phases...
- l'écran d'affichage fait partie de l'IHM.
- l'écran d'affichage est un écran numérique tactile, typiquement à affichage en couleurs.
- l'écran d'affichage comprend en outre la/une ou les/des touches tactiles permettant d'opérer des sélections, des validations, des réglages, des démarrages ou des arrêts de fonctionnement... Les touches tactiles sont à actionnement digital, c'est-à-dire qu'elles sont activées lorsque l'utilisateur appuie dessus avec son doigt, typiquement son index.
- l'écran d'affichage est configuré pour opérer un affichage d'informations sous forme de caractères alphanumériques, de représentations graphiques (e.g. graphes, courbes, dessins, icones, etc....), de photos, d'animations vidéo...... ou autres.
- une ligne commune de fourniture de gaz à un patient raccordée fluidiquement auxdites ligne d'insufflation et ligne d'exsufflation.
- il comprend des valves pneumatiques agencées sur la ligne d'exsufflation et sur la ligne d'insufflation et des moyens de commande pneumatiques commandant pneumatiquement lesdites valves pneumatiques.
- les valves pneumatiques agencées sur la ligne d'exsufflation sont configurées pour contrôler une mise en communication fluidique de la ligne d'exsufflation avec l'atmosphère et/ou une mise en communication fluidique de la ligne commune de fourniture de gaz avec la ligne d'exsufflation.
- les valves pneumatiques agencées sur la ligne d'insufflation sont configurées pour contrôler une mise en communication fluidique de la ligne d'insufflation avec l'atmosphère et/ou une mise en communication fluidique de la ligne d'insufflation avec la ligne commune de fourniture de gaz.
- la turbine est configurée pour délivrer de l'air.
- une (chaque) phase d'insufflation a une durée comprise entre 0.5 et 10 secondes, typiquement jusqu'à 3 à 5 secondes environ.
- une (chaque) phase d'exsufflation a une durée comprise entre 0.5 et 10 secondes, typiquement jusqu'à 3 à 5 secondes environ.
- les moyens de pilotage sont configurés pour commander la turbine de manière à opérer, i.e. déclencher, cycliquement une phase d'insufflation et une phase d'exsufflation en alternant répétitivement les phases d'insufflation et d'exsufflation.
- optionnellement, la (chaque) phase d'insufflation et la (chaque) phase d'exsufflation qui la suit sont elles-mêmes suivies par une phase de pause, i.e. située entre une phase d'exsufflation et la phase d'insufflation suivante.
- pendant la (chaque) phase de pause, la turbine est commandée par les moyens de pilotage pour délivrer une pression de pause supérieure ou égale à 0 mbar dans la ligne d'insufflation, de préférence une pression de pause entre 0 et 30 mbar, par exemple de l'ordre de 10 à 20 mbar.
- la (chaque) phase de pause a une durée de pause comprise entre 0 et 5 secondes environ, typiquement moins de 2 secondes.
- les moyens de pilotage comprennent un (micro)contrôleur numérique relié électriquement à la turbine.
- les moyens de pilotage comprennent un microprocesseur, de préférence porté par une carte électronique.
- la turbine comprend un moteur électrique fonctionnant grâce à un courant électrique.
- la turbine comprend un moteur électrique entraînant une roue à ailettes agencée dans le compartiment interne d'une volute.
- pendant une phase d'insufflation et/ou une phase de pause éventuelle, la turbine délivre de l'air sous pression (i.e. > à la pression atmosphérique) dans la ligne d'insufflation raccordée fluidiquement à la sortie de gaz de la turbine, c'est-à-dire que la turbine génère une pression positive (P+) dans ladite ligne d'insufflation. L'air sous pression y chemine en direction de la ligne commune de fourniture de gaz et donc du patient.
- pendant une (chaque) phase de pause éventuelle, la turbine génère une pression positive (P+) comprise entre 0 et 30 mbar (pression relative par rapport à la pression atmosphérique).
- pendant une (chaque) phase d'exsufflation, la turbine génère une pression négative (P-) du côté de l'entrée de gaz de la turbine, c'est-à-dire une dépression (i.e. une pression inférieure ou égale à la pression atmosphérique), dans la ligne d'exsufflation et dans la ligne commune de fourniture de gaz qui est fluidiquement raccordée à la ligne d'exsufflation. Ceci permet de mettre les voies respiratoires du patient en dépression. L'air aspiré est ensuite délivré dans la ligne d'insufflation, puis évacué vers l'atmosphère ambiante, via l'orifice de mise à l'atmosphère de la deuxième valve pneumatique.
- la turbine est commandée pour atteindre une vitesse de rotation maximale de 75 000 tours/minute, typiquement entre 10 000 et 50 000 tr/min.
- il comprend des moyens d'alimentation en courant électrique alimentant en courant électrique au moins la turbine motorisée, l'IHM et les moyens de pilotage et, éventuellement, la pompe ou le compresseur.
- les moyens d'alimentation en courant électrique comprennent au moins une batterie, de préférence rechargeable, et/ou une prise électrique et un cordon électrique de raccordement au secteur (e.g. 110/230 V), et éventuellement un transformateur de courant.
- le circuit de gaz, en particulier la ligne commune de fourniture de gaz, est relié fluidiquement au patient via une interface respiratoire, tel un masque respiratoire, par exemple un masque bucco-nasal, ou un embout buccal.
- la ligne commune de fourniture de gaz est reliée fluidiquement à l'interface respiratoire par l'intermédiaire d'un tuyau flexible ou analogue.

L'appareil à toux de l'invention est adapté au traitement des patients adultes, y compris les personnes âgées, mais aussi pédiatriques, c'est-à-dire les enfants ou les adolescents.

L'invention est définie par les revendications annexées.

L'invention va maintenant être mieux comprise grâce à la description détaillée suivante, faite à titre illustratif mais non limitatif, en référence aux figures annexées parmi lesquelles :
- [Fig. 1] est un schéma d'une architecture interne d'un appareil d'insufflation et d'exsufflation de gaz,
- [Fig. 2] schématise un mode de réalisation de l'écran d'affichage d'un appareil à toux selon l'invention avant le démarrage d'un traitement,
- [Fig. 3] schématise l'écran d'affichage de [Fig. 2] lors d'une insufflation de gaz en mode INEX, et
- [Fig. 4] schématise l'écran d'affichage de [Fig. 2] lors d'une exsufflation de gaz en mode INEX.

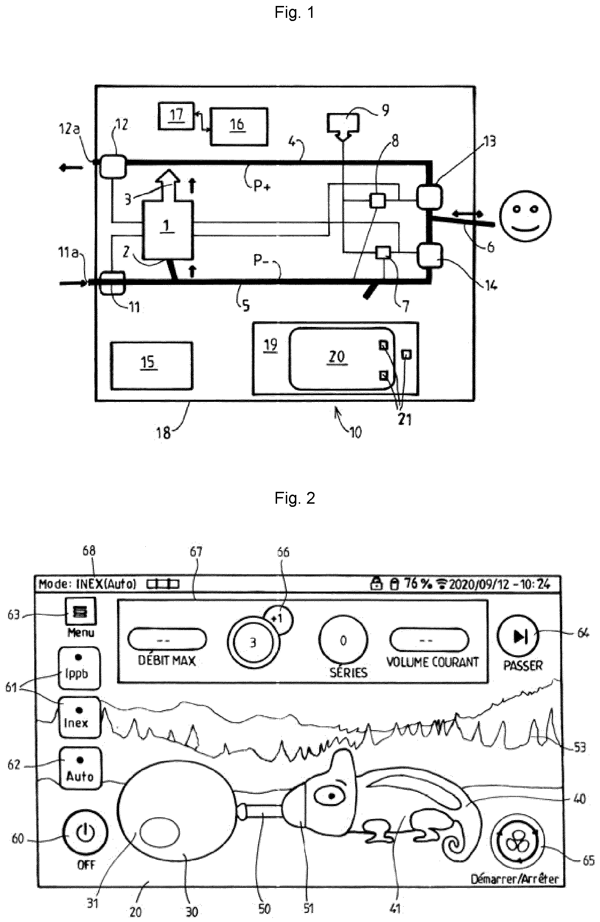

[Fig. 1] schématise une architecture interne d'un appareil d'assistance à la toux 10 ou appareil à toux, tel celui de l'invention, permettant de réaliser des insufflations et des exsufflations de gaz à un patient souffrant de troubles respiratoires nécessitant une aide à l'évacuation des sécrétions pulmonaires. Cette architecture est classique et décrite en détail par EP-A-3622991 auquel on peut se reporter pour plus de détail.

Schématiquement, l'appareil à toux 10 comprend une turbine 1 motorisée, aussi appelé compresseur ou (micro)soufflante, comprenant une entrée de gaz 2 par laquelle l'air est aspiré et pénètre dans la turbine 1 et une sortie de gaz 3 par laquelle l'air, de préférence à pression positive, est expulsé et ressort de la turbine 1. La turbine 1 comprend classiquement un moteur électrique entraînant une roue à ailettes agencée dans le compartiment interne de la volute et servant à délivrer de l'air sous pression (> 1 bar).

Il est également prévu des moyens de pilotage 16 commandant notamment la turbine 1, via une liaison électrique, pour délivrer du gaz pendant au moins des phases d'insufflation et d'exsufflation.

Préférentiellement, chaque séquence d'insufflation/d'exsufflation est suivie par une phase de pause, située entre une phase d'exsufflation et la phase d'insufflation suivante, ayant une durée comprise entre 0 et 5 sec environ. Pendant la phase de pause, la turbine 1 est commandée par les moyens de pilotage 16 pour délivrer une pression gazeuse comprise entre 0 et 30 mbar dans la ligne d'insufflation 4 et la ligne commune 6.

De préférence, les moyens de pilotage 16 comprennent un ou plusieurs microprocesseurs, typiquement un contrôleur numérique relié électriquement à la turbine 1. Le contrôleur numérique peut comprendre par exemple une carte électronique à microcontrôleur mettant en œuvre un ou plusieurs algorithmes servant à piloter la turbine 1 et des moyens de mémorisation, telle une mémoire flash ou autre.

Pendant le fonctionnement de l'appareil 1, la turbine 1 alimente en air, une ligne d'insufflation 4 qui est raccordée fluidiquement à la sortie de gaz 3 de la turbine 1 et permet d'acheminer l'air expulsé par la turbine 1. Par ailleurs, une ligne d'exsufflation 5 est quant à elle raccordée fluidiquement à l'entrée de gaz 2 de la turbine 1 et permet notamment d'y acheminer l'air aspiré par la turbine 1. Les lignes d'insufflation 4 et d'exsufflation 5 sont par exemple des conduits de gaz, des passages de gaz ou analogues, agencés dans l'appareil à toux 10.

Les lignes d'insufflation 4 et d'exsufflation 5 sont par ailleurs raccordées fluidiquement à une ligne commune 6 de fourniture de gaz, tel un conduit de gaz ou analogue, qui est reliée fluidiquement à un patient, par exemple via un tuyau flexible relié fluidiquement à une interface respiratoire, tel qu'un masque respiratoire ou analogue, de sorte d'alimenter les voies respiratoires du patient, notamment ses poumons, en air sous pression pendant les phases d'insufflation et, à l'inverse, d'en extraire le gaz pendant les phases d'exsufflation et à l'aider ainsi à expulser ses sécrétions pulmonaires ou bronchiques.

Les lignes d'insufflation 4 et d'exsufflation 5 comprennent plusieurs valves pneumatiques 11, 12, 13, 14 permettant de contrôler les flux gazeux pendant les phases d'insufflation et d'exsufflation successives, et optionnellement pendant les phases de pause et/ou transitoires.

Schématiquement, une première 11 et une quatrième 14 valves pneumatiques sont agencées sur la ligne d'exsufflation 5. La première valve pneumatique 11 contrôle la mise en communication fluidique de la ligne d'exsufflation 5 avec l'atmosphère ambiante, via un orifice d'entrée 11a, pour permettre à de l'air atmosphérique de d'y pénétrer et d'alimenter la turbine 1, pendant les phases d'insufflation, alors que la quatrième valve pneumatique 14 contrôle la mise en communication fluidique de la ligne commune 6 de fourniture de gaz avec la ligne d'exsufflation 5, pendant les phases d'exsufflation.

Par ailleurs, une deuxième 12 et une troisième 13 valves pneumatiques sont agencées sur la ligne d'insufflation 4. La deuxième valve pneumatique 12 contrôle la mise en communication fluidique de la ligne d'insufflation 4 avec l'atmosphère, via un orifice d'évacuation de gaz 12a pour évacuer le gaz sous pression, pendant les phases d'exsufflation, comme expliqué ci-après, alors que la troisième valve pneumatique 13 contrôle la mise en communication fluidique de la ligne d'insufflation 4 avec la ligne commune 6 de fourniture de gaz, pour alimenter le patient en air, pendant les phases d'insufflation.

Ces valves pneumatiques 11-14 sont elles-mêmes pilotées pneumatiquement par des moyens de commande pneumatiques 7, 8 comprenant des première et seconde électrovannes pneumatiques 7, 8, à savoir des électrovannes miniatures à faible consommation de pic (i.e. < 10 Watt) qui sont pilotées électriquement, via une ou plusieurs liaisons électriques, par les moyens de pilotage 16 de l'appareil à toux 10, en fonction de la phase d'insufflation ou d'exsufflation à opérer, et/ou des éventuelles phases de pause.

Les première et seconde électrovannes pneumatiques 7, 8 sont par ailleurs reliées pneumatiquement à au moins une ligne de pilotage pneumatique, c'est-à-dire au moins une ligne d'amenée de pression positive, par exemple une conduite de gaz qui se ramifie ou plusieurs conduites, reliant ces première et seconde électrovannes pneumatiques 7, 8 à une source de pression positive 9, i.e. une source de gaz sous pression, tel qu'une micro-pompe ou micro-compresseur additionnel.

En outre, les première et seconde électrovannes 7, 8 sont aussi connectées fluidiquement à une source de pression dite « négative », appelée simplement source de pression négative, via au moins une ligne d'amenée de pression négative, par exemple à la ligne d'exsufflation 5 où règne une pression négative (P-) pendant les phases d'exsufflation.

Le fonctionnement et la structure de ces valves pneumatiques et électrovannes sont détaillés dans EP-A-3622991, auquel on peut se reporter pour plus de détail.

Par exemple, en mode INEX, l'insufflation de gaz par la turbine 1 s'effectue à pression positive haute assez élevée, par exemple de l'ordre de 30 bar, pendant une durée d'insufflation courte, typiquement au maximum de l'ordre de 3 sec, suivi d'une exsufflation de gaz par mise en dépression (i.e. pression négative), par exemple de l'ordre de -30 mbar, des poumons et de la cage thoracique du patient pour provoquer la mobilisation et l'expulsion/extraction des sécrétions pulmonaires et/ou bronchiques du patient.

Autrement dit, pendant une phase d'insufflation, la turbine 1 génère une pression positive (P+) du côté de la sortie 3 de gaz de la turbine 1, c'est-à-dire une surpression, dans la ligne d'insufflation 4 et dans la ligne commune 6 de fourniture de gaz qui est fluidiquement raccordée à la ligne d'insufflation 5, pendant une durée d'insufflation courte, ce qui met le tractus respiratoire du patient en surpression du fait de l'apport de gaz, i.e. d'air, sous pression.

Ensuite, pendant la phase d'exsufflation qui suit la phase d'insufflation, la turbine 1 génère une pression négative (P-) subite du côté de l'entrée 2 de gaz de la turbine 1, c'est-à-dire un vide (dépression), dans la ligne d'exsufflation 5 et dans la ligne commune 6 de fourniture de gaz qui est fluidiquement raccordée à la ligne d'exsufflation 5, ce qui met le tractus respiratoire du patient en dépression, pendant une durée d'exsufflation également courte, c'est-à-dire de moins de 3 secondes, en engendrant alors l'expulsion/extraction des sécrétions pulmonaires et/ou bronchiques du patient.

L'air aspiré est ensuite délivré dans la ligne d'insufflation 5, puis évacué vers l'atmosphère ambiante, via l'orifice de mise à l'atmosphère 12a de la deuxième valve pneumatique 12. Par exemple, la turbine 1 génère une pression négative (P-) pouvant atteindre environ -70 mbar, par exemple de l'ordre de -30 mbar.

La pression positive haute (P+), la pression négative (P-) et les durées d'insufflation et/ou d'exsufflation et tous les autres paramètres ventilatoires sont réglables. Ils sont réglés par un personnel soignant puis mémorisés sous forme de préréglages, c'est-à-dire d'ensembles de paramètres respiratoires correspondant aux modes ventilatoires INEX et IPPB.

Pendant les phases d'insufflation et d'exsufflation, la turbine 1 est commandée par les moyens de pilotage 16. La vitesse de rotation maximale de la turbine peut atteindre environ 75 000 tours/minute, typiquement entre 10 000 et 50 000 tr/min.

Les éléments de l'appareil à toux 10 montrés sur la [Fig. 1] peuvent être agencés dans une carcasse 18 ou coque externe rigide, par exemple une carcasse en polymère ou analogue.

Une interface homme-machine 19 ou IHM, agencée sur la carcasse 18, permet à l'utilisateur de rentrer des valeurs de consigne dans l'appareil à toux 10, d'opérer des sélections ou des choix...

L'IHM 19 comprend un écran tactile 20, de préférence à affichage en couleurs, et des touches 21 de sélection ou autres, notamment numériques tactiles s'affichant sur l'écran tactile 20, i.e. écran digital.

Des moyens d'alimentation 15 en courant électrique (non montrés) alimentent en courant électrique les différents composants de l'appareil à toux 10 nécessitant de l'être, à savoir notamment la turbine 1 motorisée, les moyens de pilotage 16, l'IHM 19, l'écran d'affichage 20... Les moyens d'alimentation 15 en courant électrique comprennent par exemple une (ou plusieurs) batterie, de préférence rechargeable, et/ou une prise électrique et un cordon électrique (non montré) de raccordement au secteur, e.g. 110/230 V, avec ou sans transformateur de courant.

D'une manière générale, un appareil à toux 10 selon la présente invention est destiné à être utilisé pour opérer des insufflations et des exsufflations de gaz (i.e. d'air) à des patients incapables de gérer seuls leurs sécrétions de sorte de leur fournir des insufflations et exsufflations de gaz, que ces patients soient des patients adultes ou pédiatriques. Il peut être utilisé à domicile ou à l'hôpital, avec des interfaces respiratoires invasives (e.g. sondes trachéales) ou non-invasives (e.g. masques).

L'appareil à toux 10 est préférentiellement suffisamment léger et compact pour être facilement transportable.

Les moyens de pilotage 16 sont aussi configurés pour commander l'affichage d'informations de toute nature, à savoir des caractères alphanumériques, des icônes, des représentations graphiques, des courbes ou autres, sur l'écran d'affichage 20 de l'IHM 19, lequel opère alors un affichage de ces informations de manière à assister, informer ou autre, l'utilisateur.

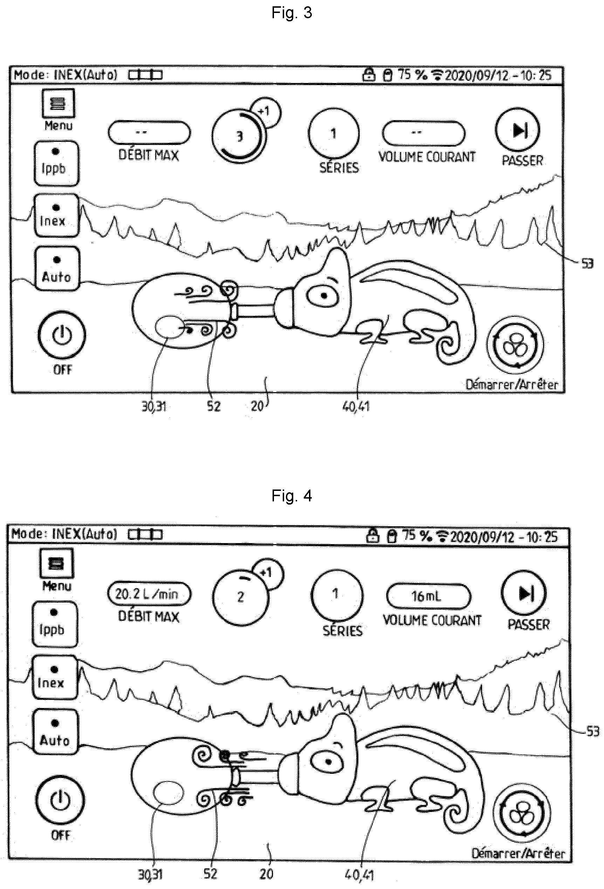

Selon la présente invention, afin de permettre l'utilisation de l'appareil à toux 10 par une personne moins formée qu'un kinésithérapeute, par exemple une auxiliaire de vie, une personne de l'entourage familial du patient... afin de pouvoir déclencher chez le patient, une séance d'utilisation de l'appareil à toux 10, au moment le plus opportun pour le patient considéré, sans avoir à faire venir spécialement un professionnel de santé qualifié de type kinésithérapeute ou analogue, et à attendre son passage, les moyens de pilotage 16 sont configurés pour commander un affichage simultané, sur l'écran d'affichage 20 tactile, une animation vidéo mettant en œuvre une première représentation graphique 30 symbolisant la source de fourniture de gaz, i.e. au moins les phases d'insufflation et d'exsufflation de gaz et une seconde représentation graphique 40 symbolisant le patient qui doit être traité, et dans laquelle la forme, l'aspect et/ou les dimensions des première et/ou seconde représentations graphiques 30, 40 affichées sur l'écran d'affichage 20 varient en corrélation avec les phases d'insufflation et d'exsufflation de gaz par la turbine 2 de l'appareil à toux 10, voire aussi toute phase de pause éventuelle, comme illustré sur les [Fig. 2] à [Fig. 4]. En particulier, les dimensions des première et seconde représentations graphiques 30, 40 affichées sur l'écran d'affichage 20 varient en corrélation avec les phases d'insufflation et d'exsufflation de gaz, et inversement l'une par rapport à l'autre.

Dans le mode de réalisation illustré sur ces [Fig. 2] à [Fig. 4], concernant le mode INEX, la première représentation graphique 30 symbolisant les phases d'insufflation et d'exsufflation de gaz de l'appareil à toux 10 est représentée par un contenant à gaz, à savoir ici un ballon de baudruche 31, alors que la seconde représentation graphique 40 symbolisant le patient, en particulier les phases inspiratoire et expiratoire dudit patient pendant le traitement, est représentée par un animal, à savoir ici un caméléon 41. Bien entendu, tout autre contenant et/ou autre animal ou personnage peuvent convenir.

Plus précisément, afin d'assister une personne n'ayant pas ou peu de connaissances médicales, par exemple une auxiliaire de vie ou une personne de l'entourage familial du patient (les aidants), dans la mise en œuvre d'un appareil à toux 10 selon l'invention de manière à opérer des insufflations et des exsufflations de gaz à un patient souffrant de troubles respiratoires nécessitant une aide à l'évacuation des sécrétions pulmonaires, les moyens de pilotage 16 commandent l'affichage sur l'écran d'affichage 20, dans le mode de réalisation proposé en exemple, à savoir ici en mode INEX, de la vidéo d'animation mettant en scène ici un ballon de baudruche 31, en tant que première représentation graphique 30 symbolisant les phases d'insufflation et d'exsufflation de gaz opérées au moyen de l'appareil à toux 10 et, par ailleurs, un animal, à savoir ici un caméléon 41, en tant que seconde représentation graphique 40 symbolisant le patient et ses phases respiratoires (i.e. inspiratoire et expiratoire), et préférentiellement une phase de pause optionnelle.

Dans cette animation vidéo, le ballon de baudruche 31 est relié au caméléon 41 par des représentations graphiques additionnelles 50, 51, 52 s'affichant à l'écran 20, à savoir un tuyau 50, un masque respiratoire 51 et un flux respiratoire 52 symbolisant la circulation de gaz entre le ballon de baudruche 31 et le caméléon 41. Bien entendu, d'autres représentations graphiques 30, 31, 40, 41, 50, 51 sont possibles.

On voit aussi que l'écran 20 comprend un fond d'écran 53 comprenant un décor ou analogue, à savoir ici une représentation de paysage, le ballon de baudruche 31 et caméléon 41, ainsi que les représentations graphiques additionnelles 50, 51, 52, venant se superposer audit paysage afin de créer une ambiance agréable et ludique pour l'utilisateur.

L'appareil à toux 10 comprend par ailleurs des moyens de mémorisation 17, telle qu'une carte mémoire, par exemple une EEPROM ou directement dans un exécutable (i.e. programme de processeur) ou autre, pour mémoriser la vidéo d'animation (i.e. animation vidéo) ou d'autres paramètres, en particulier les préréglages pour les modes INEX ou IPPB.

Les moyens de pilotage 16 sont donc configurés pour aller retrouver dans les moyens de mémorisation 17 et ensuite afficher à l'écran 20, l'animation vidéo au début d'un traitement, en particulier au démarrage d'une phase d'insufflation de gaz, après sélection du mode de ventilation désiré, à savoir ici le mode INEX, et ensuite activation (i.e. appui digital) par l'utilisateur d'une touche tactile 65 de démarrage/arrêt de traitement, comme détaillé ci-après.

Par ailleurs, les moyens de pilotage 16 sont aussi configurés pour aller retrouver au sein des moyens de mémorisation 17, les préréglages des modes ventilatoires INEX ou IPPB et ensuite les appliquer, c'est-à-dire commander par exemple la turbine 2, après sélection par l'utilisateur du mode désiré, typiquement après appui sur une touche de sélection de mode 61, comme expliqué ci-après.

Ainsi, on voit sur [Fig. 2], qui montre l'écran 20 avant le démarrage d'un traitement, que l'écran d'affichage 20 de l'IHM 19 comprend d'autres éléments, tels des moyens d'activation, de sélection ou analogue, en particulier des touches de sélection, notamment de préréglages permettent de sélectionner/activer, i.e. d'utiliser, des préréglages (appelées '*presets*' en anglais) préenregistrés, i.e. mémorisés, c'est-à-dire les réglages particuliers (e.g. pression, durées, pause, etc...) des modes ventilatoires INEX et IPPB, qui sont donc adaptés à certaines situations cliniques données, par exemple pour la nuit, pour le matin...

Par exemple, l'écran d'affichage 20 de l'IHM 19 comprend dans le mode de réalisation proposé :
- une touche tactile 60 de mise en route ou arrêt (OFF) de l'appareil.
- des touches tactiles 61 pour régler ou sélectionner des préréglages mémorisés adaptés aux modes de ventilation INEX et IPPB, par exemple pour régler les durées d'insufflation et d'exsufflation, les pressions P+ et P- en mode INEX, les temps d'inspiration et d'expiration, etc... Ces préréglages correspondent à des ensembles de paramètres ventilatoires ayant été choisis par un personnel soignant, tel un médecin ou un kinésithérapeute, puis mémorisés dans les moyens de mémorisation de l'appareil 10.
- une touche tactile 62 pour par exemple sélectionner un préréglage (preset) pour une ventilation en mode INEX AUTO, c'est-à-dire en mode INEX avec sous-mode de fonctionnement automatique (AUTO).
- une touche tactile 66 permettant de sélection ou rajouter un cycle de ventilation supplémentaire, si besoin est.
- une touche tactile 64 (PASSER) pour stopper un cycle de ventilation en cours, c'est-à-dire avant la fin du cycle.
- une touche tactile 65 de démarrage/arrêt de traitement pour démarrer et/ou stopper le fonctionnement de la turbine 2, et donc pour commencer ou arrêter des insufflations et exsufflations de gaz, en particulier pour commencer ou arrêter une séance de traitement du patient.
- une touche tactile 63 pour accéder à différents menus ou sélections.

L'écran d'affichage 20 permet aussi d'afficher, par exemple au sein d'une fenêtre 67 située en haut d'écran 20, un débit maximal de gaz (en L/min par exemple), un volume courant de gaz (en mL par exemple) et d'autres informations ou paramètres, tel que le nombre de cycles et/ou de séries....

Enfin, l'écran d'affichage 20 comprend aussi, dans le mode de réalisation proposé, un bandeau haut 68, dans lequel sont affichées différentes informations utiles comme le rappel du mode (ici le mode INEX par exemple) et sous-mode (AUTO) ayant été sélectionnés, une icône d'autonomie de la batterie électrique (ici 76%), une icône de transmission sans fil, par exemple en wifi, la date, l'heure.... ou autres.

[Fig. 3] schématise l'affichage sur l'écran d'affichage 20 lors d'une insufflation de gaz montrant l'animation vidéo mis en œuvre par les moyens de pilotage 16 comprenant les représentations graphiques 30, 40 d'aide au patient, en particulier le ballon de baudruche 31 et le caméléon 51 choisis dans le mode de réalisation proposé ici, au début d'une phase d'insufflation de gaz, c'est-à-dire au début du traitement d'un patient, alors que [Fig. 4] schématise l'affichage à l'écran d'affichage 20, après le démarrage d'une exsufflation de gaz, sachant que la durée d'une phase d'insufflation est par exemple de l'ordre de 1 à 3 secondes et celle d'exsufflation est par exemple de l'ordre de 1 à 3 secondes.

Comme on peut le voir, sur la [Fig. 2], le ballon de baudruche 31 est représenté (i.e. forme/aspect) totalement gonflé, donc avec des dimensions maximales, alors qu'à l'inverse, le caméléon 41 est représenté (i.e. forme/aspect) avec les poumons (quasiment) vides de gaz, donc avec des dimensions « minimales ».

Au début d'une phase d'insufflation de gaz, i.e. d'air, les moyens de pilotage 16 commandent l'affichage sur l'écran 20 de l'animation vidéo, après appui par l'utilisateur sur une touche tactile 65 de démarrage/arrêt de traitement, de manière à montrer le ballon de baudruche 31 se dégonflant progressivement, c'est-à-dire que les dimensions du ballon et son aspect/sa forme se réduisent à l'écran 20, mimant ainsi une sortie de gaz 52 du ballon 31 sur la [Fig. 3], et par ailleurs le caméléon 41 représentant le patient qui respire, se gonflant progressivement et simultanément, c'est-à-dire que le caméléon 41 grossit/se gonfle (i.e. comme le font les poumons du patient), alors que dans le même temps, le ballon de baudruche 31 se dégonfle mimant ainsi une entrée de gaz 52 dans les poumons du patient.

A l'inverse, lors d'une phase d'exsufflation de gaz, l'animation vidéo montre l'inverse, à savoir le ballon de baudruche 31 qui se gonfle alors que simultanément le caméléon 41, i.e. représentant le patient pendant son traitement, se dégonfle mimant ainsi une sortie de gaz 52 des poumons du patient, comme schématisé en [Fig. 4].

Selon les phases d'insufflation et d'exsufflation de gaz, les dimensions du caméléon 41 et celles du ballon de baudruche 31 varient donc de manière corrélée, i.e. synchronisée, et inversement les unes aux autres.

Le déroulé de l'affichage de la vidéo d'animation est synchronisé avec les durées d'insufflation et d'exsufflation, lors des phases d'insufflation ou d'exsufflation de gaz en mode INEX. Bien entendu, les durées d'insufflation et d'exsufflation et les autres paramètres du mode INEX sont réglables, i.e. paramétrables, par un personnel soignant, tel un médecin par exemple, et mémorisés par les moyens de mémorisation.

On voit aussi le flux gazeux 52 allant du ballon 31 vers le caméléon 41 (cf. [Fig. 3]) ou inversement (cf. [Fig. 4]) pour schématiser la circulation du gaz en direction du patient ou dans le sens opposé.

Pendant une éventuelle phase de pause (non montrée) suivant une phase d'exsufflation de gaz, l'animation vidéo montre un ballon de baudruche 31 et un caméléon 41 non-évolutifs, c'est-à-dire que leurs formes, dimensions et aspects ne changent pas à l'écran 20 étant donné qu'aucun échange gazeux ne se produit entre eux.

Autrement dit, de façon schématique, l'écran 20 affiche une animation vidéo qui montre:
- pendant chaque inspiration/insufflation (cf. [Fig. 3]), un caméléon 41 (i.e. patient) qui gonfle et un ballon 31 qui se dégonfle,
- pendant chaque expiration/l'exsufflation (cf. [Fig. 4]), un caméléon 41 (i.e. patient) qui dégonfle et un ballon 31 qui gonfle, et
- pendant chaque pause éventuelle, un caméléon 41 et un ballon 31 ne varient/changent pas.

En affichant de telles représentations 30, 40 sur l'écran 20, l'appareil à toux 10 procure une aide visuelle très utile au patient et à la personne qui l'assiste lors de la mise en œuvre de son traitement puisque le patient n'a qu'à calquer ses phases inspiratoires et expiratoires sur les gonflages/dégonflages des représentations 30, 40 présentées à l'écran 20, i.e. caméléon 41 et ballon 31, à savoir ; inspirer pendant le temps où le caméléon 41 gonfle et expirer pendant le temps où le caméléon 41 dégonfle.

L'animation vidéo mise en œuvre sur l'écran 20 est ici une véritable solution technique au problème susmentionné. En effet, en procédant ainsi, c'est-à-dire en calquant ses phases inspiratoires et expiratoires, sur les phases d'insufflation et d'exsufflation de gaz par la turbine 2 tel que montré par l'animation vidéo, le patient est assuré de bien réaliser son traitement, c'est-à-dire d'obtenir un traitement efficace (e.g. expulsions des sécrétions, mucus ou autres), et ce, sans nécessiter la présence sur place d'un personnel soignant qualifié, de type kinésithérapeute ou analogue.

Lorsque le patient a bien réalisé son traitement, c'est-à-dire à bien calqué sa respiration sur les phases d'insufflations et d'exsufflation de l'appareil à toux, les moyens de pilotage 16 peuvent être en outre aussi configurés pour afficher sur l'écran d'affichage 20, une animation vidéo additionnelle symbolisant cette bonne réalisation de son traitement par le patient, par exemple une animation vidéo additionnelle du type pluie de confetti ou une récompense comme une coupe ou un trophée, ou autre.

L'appareil d'assistance à la toux ou appareil à toux selon l'invention permet d'opérer efficacement des insufflations et des exsufflations de gaz à un patient souffrant de troubles respiratoires nécessitant une aide à l'évacuation des sécrétions pulmonaires, même lorsque le patient est assisté par une personne n'ayant pas ou peu de connaissances médicales, par exemple une auxiliaire de vie ou une personne de l'entourage familial du patient, encore appelés un « aidant », puisqu'il lui suffit de calquer sa respiration sur l'animation vidéo qui s'affiche à l'écran.

## Revendications

1. Appareil à toux (10) pour opérer des insufflations et exsufflations de gaz à un patient, comprenant :
- une turbine, un compresseur ou une (micro)soufflante (1) en communication fluidique avec un circuit de gaz (4, 5, 6) comprenant une ligne d'insufflation (4) et une ligne d'exsufflation (5),
- un écran d'affichage (20),
- et des moyens de pilotage (16) comprenant un microprocesseur, configurés pour commander la turbine, le compresseur ou la (micro)soufflante (1) et un affichage sur l'écran d'affichage (20), et dans lequel les moyens de pilotage (16) sont en outre configurés pour commander un affichage sur l'écran d'affichage (20), d'une animation vidéo mettant en œuvre une première représentation graphique (30) symbolisant au moins les phases d'insufflation et d'exsufflation de gaz et une seconde représentation graphique (40) symbolisant le patient, où
- il comprend en outre des moyens de mémorisation configurés pour mémoriser la vidéo d'animation,
- les moyens de pilotage (16) sont configurés pour commander la turbine, le compresseur ou la (micro)soufflante (1) de manière à déclencher cycliquement une phase d'insufflation et une phase d'exsufflation, et
- le déroulé de l'affichage de la vidéo d'animation est synchronisé avec les durées d'insufflation et d'exsufflation, de sorte que les dimensions des première et seconde représentations graphiques affichées varient de manière inverse les unes des autres, ou la forme ou l'aspect desdites première et seconde représentations graphiques (30, 40) varient, en corrélation avec lesdites phases d'insufflation et d'exsufflation de gaz,
- et les moyens de pilotage (16) sont en outre configurés pour commander la turbine, le compresseur ou la (micro)soufflante (1) pour délivrer une pression de pause supérieure ou égale à 0 mbar, pendant une phase de pause séparant une phase d'exsufflation de la phase d'insufflation suivante.

2. Appareil selon la revendication 1, **caractérisé en ce que** les moyens de pilotage (16) sont configurés pour afficher sur l'écran d'affichage (20), une animation vidéo dans laquelle la forme, l'aspect et/ou les dimensions des première et seconde représentations graphiques (30, 40) affichées sur l'écran d'affichage (20) varient de manière synchronisée l'une à l'autre.

3. Appareil selon l'une des revendications 1 ou 2, **caractérisé en ce que** les variations de dimensions de la première représentation graphique et de la seconde représentation graphique se font de manière progressive entre le début et la fin des phases d'insufflation et d'exsufflation de gaz et/ou des phases inspiratoire et expiratoire du patient.

4. Appareil selon l'une des revendications 1 à 3, **caractérisé en ce que** :
- la première représentation graphique (30) représente ou comprend un objet contenant du gaz, en particulier un ballon de baudruche (31) et
- la seconde représentation graphique (40) représente ou comprend un animal ou un personnage, en particulier un caméléon (41).

5. Appareil selon la revendication 1, **caractérisé en ce que** les moyens de pilotage (16) sont configurés pour commander la turbine, compresseur ou (micro)soufflante (1) de manière à déclencher cycliquement et en alternance des phases d'insufflation et d'exsufflation en mode INEX avec insufflation de gaz à une pression positive d'au moins 5 mbar suivie d'une exsufflation de gaz par mise en dépression.

6. Appareil selon la revendication 1, **caractérisé en ce qu'**il comprend des moyens de sélection de mode de ventilation comprenant au moins une touche tactile affichée sur l'écran d'affichage (20).

7. Appareil selon la revendication 6, **caractérisé en ce que** les moyens de sélection permettent de choisir un mode de ventilation de type INEX.

8. Appareil selon la revendication 6 ou 7, **caractérisé en ce que** les moyens de sélection de mode de ventilation sont configurés pour sélectionner un ensemble de paramètres ou préréglages ventilatoires adapté à la mise en œuvre d'une ventilation en mode de ventilation INEX.

9. Appareil selon la revendication 1, **caractérisé en ce que** l'écran d'affichage (20) est un écran tactile configuré pour afficher la vidéo d'animation en couleurs.

10. Appareil selon la revendication 1 ou 9, **caractérisé en ce que** l'écran d'affichage (20) est configuré pour afficher en outre un débit maximal de gaz ou un volume courant de gaz.

11. Appareil selon la revendication 1, **caractérisé en ce que** les moyens de pilotage (16) sont configurés pour commander la turbine, le compresseur ou la (micro)soufflante (1) pour délivrer une pression de pause comprise entre 0 et 30 mbar.

12. Appareil selon l'une des revendications 1 ou 11, **caractérisé en ce que** les moyens de pilotage (16) sont configurés pour opérer, pendant une phase de pause, un affichage non-évolutif de l'animation vidéo dans lequel les formes, dimensions et aspects des première et seconde représentations graphiques (30, 40) affichées ne changent pas sur l'écran d'affichage (20).

13. Appareil selon la revendication 1, **caractérisé en ce que** les ligne d'insufflation (4) et d'exsufflation (5) du circuit de gaz (4, 5, 6) comprennent plusieurs valves pneumatiques (11, 12, 13, 14) permettant de contrôler les flux gazeux pendant les phases d'insufflation et d'exsufflation successives, et pendant les phases de pause.

14. Appareil selon la revendication 1, **caractérisé en ce que** chaque phase de pause a une durée comprise entre 0 et 5 secondes.

15. Appareil selon la revendication 1, **caractérisé en ce qu'**il comprend en outre:
- des moyens de démarrage/arrêt de traitement comprenant une touche de démarrage/arrêt unique (65) commandant le démarrage et l'arrêt de la turbine, du compresseur ou de la (micro)soufflante (1), et simultanément de l'animation vidéo, et/ou
- des valves pneumatiques (12, 13) agencées sur la ligne d'insufflation (4) et configurées pour contrôler une mise en communication fluidique de la ligne d'insufflation (4) avec une ligne commune (6) de fourniture de gaz raccordée fluidiquement auxdites ligne d'insufflation (4) et ligne d'exsufflation (5).

## Patentansprüche

1. Ein Hustenassistenzgerät (10) zum Durchführen von Gasinsufflationen und -exsufflationen bei einem Patienten, umfassend:
- eine Turbine, einen Kompressor oder ein (Mikro-)Gebläse (1) in fluider Kommunikation mit einem Gaskreislauf (4, 5, 6), der eine Insufflationsleitung (4) und eine Exsufflationsleitung (5) umfasst,
- einen Anzeigebildschirm (20),
- und Steuerungsmittel (16), die einen Mikroprozessor umfassen und konfiguriert sind, um die Turbine, den Kompressor oder das (Mikro-)Gebläse (1) und eine Anzeige auf dem Anzeigebildschirm (20) zu steuern,
und wobei die Steuerungsmittel (16) ferner konfiguriert sind, um eine Anzeige auf dem Anzeigebildschirm (20) einer Videoanimation zu steuern, die eine erste grafische Darstellung (30), die mindestens die Phasen der Gasinsufflation und -exsufflation symbolisiert, und eine zweite grafische Darstellung (40), die den Patienten symbolisiert, umsetzt,
wobei
- es ferner Speichermittel umfasst, die konfiguriert sind, um die Videoanimation zu speichern,
- die Steuerungsmittel (16) konfiguriert sind, um die Turbine, den Kompressor oder das (Mikro-)Gebläse (1) so zu steuern, dass zyklisch eine Insufflationsphase und eine Exsufflationsphase ausgelöst werden, und
- der Ablauf der Anzeige der Videoanimation mit den Dauern der Insufflation und Exsufflation synchronisiert ist, so dass die Abmessungen der angezeigten ersten und zweiten grafischen Darstellungen zueinander invers variieren, oder die Form oder das Aussehen der besagten ersten und zweiten grafischen Darstellungen (30, 40) in Korrelation mit den besagten Phasen der Gasinsufflation und -exsufflation variieren,
- und die Steuerungsmittel (16) ferner konfiguriert sind, um die Turbine, den Kompressor oder das (Mikro-)Gebläse (1) zu steuern, um einen Pausendruck von mehr als oder gleich 0 mbar während einer Pausephase zu liefern, die eine Exsufflationsphase von der folgenden Insufflationsphase trennt.

2. Gerät nach Anspruch 1, **dadurch gekennzeichnet, dass** die Steuerungsmittel (16) konfiguriert sind, um auf dem Anzeigebildschirm (20) eine Videoanimation anzuzeigen, in der die Form, das Aussehen und/oder die Abmessungen der ersten und zweiten grafischen Darstellungen (30, 40), die auf dem Anzeigebildschirm (20) angezeigt werden, synchron zueinander variieren.

3. Gerät nach einem der Ansprüche 1 oder 2, **dadurch gekennzeichnet, dass** die Variationen der Abmessungen der ersten grafischen Darstellung und der zweiten grafischen Darstellung progressiv zwischen dem Anfang und dem Ende der Phasen der Gasinsufflation und -exsufflation und/oder der inspiratorischen und exspiratorischen Phasen des Patienten erfolgen.

4. Gerät nach einem der Ansprüche 1 bis 3, **dadurch gekennzeichnet, dass**:
- die erste grafische Darstellung (30) ein gasenthaltendes Objekt, insbesondere einen Luftballon (31), darstellt oder umfasst und
- die zweite grafische Darstellung (40) ein Tier oder eine Figur, insbesondere ein Chamäleon (41), darstellt oder umfasst.

5. Gerät nach Anspruch 1, **dadurch gekennzeichnet, dass** die Steuerungsmittel (16) konfiguriert sind, um die Turbine, den Kompressor oder das (Mikro-)Gebläse (1) so zu steuern, dass zyklisch und abwechselnd Insufflations- und Exsufflationsphasen im INEX-Modus mit Gasinsufflation bei einem positiven Druck von mindestens 5 mbar, gefolgt von einer Gasexsufflation durch Unterdruckerzeugung, ausgelöst werden.

6. Gerät nach Anspruch 1, **dadurch gekennzeichnet, dass** es Mittel zur Auswahl eines Beatmungsmodus umfasst, die mindestens eine auf dem Anzeigebildschirm (20) angezeigte taktile Taste umfassen.

7. Gerät nach Anspruch 6, **dadurch gekennzeichnet, dass** die Auswahlmittel die Wahl eines Beatmungsmodus vom Typ INEX ermöglichen.

8. Gerät nach Anspruch 6 oder 7, **dadurch gekennzeichnet, dass** die Mittel zur Auswahl des Beatmungsmodus konfiguriert sind, um einen Satz von Beatmungsparametern oder -voreinstellungen auszuwählen, der für die Durchführung einer Beatmung im INEX-Beatmungsmodus geeignet ist.

9. Gerät nach Anspruch 1, **dadurch gekennzeichnet, dass** der Anzeigebildschirm (20) ein taktiler Bildschirm ist, der konfiguriert ist, um die Videoanimation in Farbe anzuzeigen.

10. Gerät nach Anspruch 1 oder 9, **dadurch gekennzeichnet, dass** der Anzeigebildschirm (20) konfiguriert ist, um zusätzlich einen maximalen Gasdurchfluss oder ein Atemzugvolumen an Gas anzuzeigen.

11. Gerät nach Anspruch 1, **dadurch gekennzeichnet, dass** die Steuerungsmittel (16) konfiguriert sind, um die Turbine, den Kompressor oder das (Mikro-)Gebläse (1) zu steuern, um einen Pausendruck zwischen 0 und 30 mbar zu liefern.

12. Gerät nach einem der Ansprüche 1 oder 11, **dadurch gekennzeichnet, dass** die Steuerungsmittel (16) konfiguriert sind, um während einer Pausephase eine nicht-evolutive Anzeige der Videoanimation zu betreiben, bei der sich die Formen, Abmessungen und Aspekte der angezeigten ersten und zweiten grafischen Darstellungen (30, 40) auf dem Anzeigebildschirm (20) nicht ändern.

13. Gerät nach Anspruch 1, **dadurch gekennzeichnet, dass** die Insufflationsleitung (4) und die Exsufflationsleitung (5) des Gaskreislaufs (4, 5, 6) mehrere pneumatische Ventile (11, 12, 13, 14) umfassen, die es ermöglichen, die Gasströme während der aufeinanderfolgenden Insufflations- und Exsufflationsphasen und während der Pausephasen zu steuern.

14. Gerät nach Anspruch 1, **dadurch gekennzeichnet, dass** jede Pausephase eine Dauer zwischen 0 und 5 Sekunden hat.

15. Gerät nach Anspruch 1, **dadurch gekennzeichnet, dass** es ferner umfasst:
- Mittel zum Starten/Stoppen der Behandlung, die eine einzige Start/Stopp-Taste (65) umfassen, die den Start und das Stoppen der Turbine, des Kompressors oder des (Mikro-)Gebläses (1) und gleichzeitig der Videoanimation steuert, und/oder
- pneumatische Ventile (12, 13), die an der Insufflationsleitung (4) angeordnet und konfiguriert sind, um eine fluide Kommunikation der Insufflationsleitung (4) mit einer gemeinsamen Gaszufuhrleitung (6) zu steuern, die fluidisch mit der besagten Insufflationsleitung (4) und der Exsufflationsleitung (5) verbunden ist.

## Claims

1. A cough assist device (10) for performing gas insufflations and exsufflations on a patient, comprising:
- a turbine, a compressor or a (micro)blower (1) in fluid communication with a gas circuit (4, 5, 6) comprising an insufflation line (4) and an exsufflation line (5),
- a display screen (20),
- and pilot means (16) comprising a microprocessor, configured to control the turbine, the compressor or the (micro)blower (1) and a display on the display screen (20),
and wherein the pilot means (16) are further configured to control a display on the display screen (20) of a video animation implementing a first graphical representation (30) symbolizing at least the gas insufflation and exsufflation phases and a second graphical representation (40) symbolizing the patient,
wherein
- it further comprises storage means configured to store the video animation,
- the pilot means (16) are configured to control the turbine, the compressor or the (micro)blower (1) so as to cyclically trigger an insufflation phase and an exsufflation phase, and
- the display sequence of the video animation is synchronized with the durations of insufflation and exsufflation, such that the dimensions of the first and second displayed graphical representations vary inversely to one another, or the shape or aspect of said first and second graphical representations (30, 40) vary, in correlation with said gas insufflation and exsufflation phases,
- and the pilot means (16) are further configured to control the turbine, the compressor or the (micro)blower (1) to deliver a pause pressure greater than or equal to 0 mbar, during a pause phase separating an exsufflation phase from the following insufflation phase.

2. The device according to claim 1, **characterized in that** the pilot means (16) are configured to display on the display screen (20), a video animation in which the shape, the aspect and/or the dimensions of the first and second graphical representations (30, 40) displayed on the display screen (20) vary in a synchronized manner with respect to each other.

3. The device according to one of claims 1 or 2, **characterized in that** the variations in dimensions of the first graphical representation and of the second graphical representation occur progressively between the beginning and the end of the gas insufflation and exsufflation phases and/or of the inspiratory and expiratory phases of the patient.

4. The device according to one of claims 1 to 3, **characterized in that**:
- the first graphical representation (30) represents or comprises an object containing gas, in particular a party balloon (31) and
- the second graphical representation (40) represents or comprises an animal or a character, in particular a chameleon (41).

5. The device according to claim 1, **characterized in that** the pilot means (16) are configured to control the turbine, compressor or (micro)blower (1) so as to trigger cyclically and in alternation insufflation and exsufflation phases in INEX mode with gas insufflation at a positive pressure of at least 5 mbar followed by a gas exsufflation by creating a depression.

6. The device according to claim 1, **characterized in that** it comprises means for selecting a ventilation mode comprising at least one tactile key displayed on the display screen (20).

7. The device according to claim 6, **characterized in that** the selection means allow choosing a ventilation mode of INEX type.

8. The device according to claim 6 or 7, **characterized in that** the ventilation mode selection means are configured to select a set of ventilatory parameters or presettings adapted to the implementation of a ventilation in INEX ventilation mode.

9. The device according to claim 1, **characterized in that** the display screen (20) is a tactile screen configured to display the video animation in color.

10. The device according to claim 1 or 9, **characterized in that** the display screen (20) is configured to further display a maximum gas flow rate or a tidal volume of gas.

11. The device according to claim 1, **characterized in that** the pilot means (16) are configured to control the turbine, the compressor or the (micro)blower (1) to deliver a pause pressure comprised between 0 and 30 mbar.

12. The device according to one of claims 1 or 11, **characterized in that** the pilot means (16) are configured to operate, during a pause phase, a non-evolving display of the video animation in which the shapes, dimensions and aspects of the first and second displayed graphical representations (30, 40) do not change on the display screen (20).

13. The device according to claim 1, **characterized in that** the insufflation line (4) and exsufflation line (5) of the gas circuit (4, 5, 6) comprise several pneumatic valves (11, 12, 13, 14) allowing to control the gas flows during the successive insufflation and exsufflation phases, and during the pause phases.

14. The device according to claim 1, **characterized in that** each pause phase has a duration comprised between 0 and 5 seconds.

15. The device according to claim 1, **characterized in that** it further comprises:
- treatment start/stop means comprising a single start/stop key (65) controlling the start and stop of the turbine, the compressor or the (micro)blower (1), and simultaneously of the video animation, and/or
- pneumatic valves (12, 13) arranged on the insufflation line (4) and configured to control a fluid communication of the insufflation line (4) with a common gas supply line (6) fluidly connected to said insufflation line (4) and exsufflation line (5).
